# EUROPEAN PATENT SPECIFICATION

(11) **EP 2 827 893 B1**
(45) Date of publication and mention of the grant of the patent: **29.11.2017**
(21) Application number: 13712662.9
(22) Date of filing: 15.03.2013
(51) Int. Cl.: A61K 39/145, C07K 14/005, C12N 7/00, C12N 15/82

(54) **VIRUS-LIKE PARTICLES COMPRISING A MATRIX PROTEIN FROM A PLANT ENVELOPED VIRUS AND USES THEREOF**
VIRUSÄHNLICHE PARTIKEL, DIE EIN MATRIXPROTEIN EINES UMHÜLLTEN PFLANZENVIRUS UMFASSEN UND DEREN VERWENDUNG
PARTICULES PSEUDO-VIRALES COMPRENANT UNE PROTÉINE MATRICIELLE D'UN VIRUS ENVELOPPÉ DE PLANTES ET LEUR UTILISATIONS

(30) Priority: 22.03.2012 US 201261614141 P
(43) Date of publication of application: 28.01.2015
(73) Proprietor: Fraunhofer USA, Inc., Newark, DE 19711-5449 (US)
(72) Inventor: PROKHNEVSKY, Alexei, Wilmington, DE 19808 (US); YUSIBOV, Vidadi, Havertown, PA 19083 (US)
(74) Representative: RatnerPrestia
(86) International application number: PCT/US2013/032096
(87) International publication number: WO 2013/142329

(56) References cited:
- WO-A1-2008/058396
- WO-A1-2011/035004

### SEQUENCE LISTING

<110> Fraunhofer USA, Inc.
<120> ENVELOPED VIRUS-LIKE PARTICLES AND USES THEREOF
<130> FCMB-106WO
<150> US 61/614,141
   <151> 2012-03-22
<160> 4
<170> PatentIn version 3.5
<210> 1
   <211> 581
   <212> PRT
   <213> Avian influenza virus
<400> 1
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala Asp Gln
               20                  25                  30
   Ile Cys Ile Gly Tyr His Ala Asn Asn Ser Thr Glu Gln Val Asp Thr
           35                  40                  45
   Ile Met Glu Lys Asn Val Thr Val Thr His Ala Gln Asp Ile Leu Glu
       50                  55                  60
   Lys Thr His Asn Gly Lys Leu Cys Asp Leu Asp Gly Val Lys Pro Leu
   65                  70                  75                  80
   Ile Leu Arg Asp Cys Ser Val Ala Gly Trp Leu Leu Gly Asn Pro Met
                   85                  90                  95
   Cys Asp Glu Phe Ile Asn Val Pro Glu Trp Ser Tyr Ile Val Glu Lys
               100                 105                 110
   Ala Asn Pro Thr Asn Asp Leu Cys Tyr Pro Gly Ser Phe Asn Asp Tyr
           115                 120                 125
   Glu Glu Leu Lys His Leu Leu Ser Arg Ile Asn His Phe Glu Lys Ile
       130                 135                 140
   Leu Ile Ile Pro Lys Ser Ser Trp Ser Asp His Glu Ala Ser Ser Gly
   145                 150                 155                 160
   Val Ser Ser Ala Cys Pro Tyr Leu Gly Ser Pro Ser Phe Phe Arg Asn
   165 170 175
   Val Val Trp Leu Ile Lys Lys Asn Ser Thr Tyr Pro Thr Ile Lys Lys
   180 185 190
   Ser Tyr Asn Asn Thr Asn Gln Glu Asp Leu Leu Val Leu Trp Gly Ile
   195 200 205
   His Pro Asn Asp Ala Ala Glu Gln Thr Arg Leu Tyr Gln Asn Pro Thr
   210 215 220
   Thr Tyr Ile Ser Ile Gly Thr Ser Thr Leu Asn Gln Arg Leu Val Pro
   225 230 235 240
   Lys Ile Ala Thr Arg Ser Lys Val Asn Gly Gln Ser Gly Arg Met Glu
   245 250 255
   Phe Phe Trp Thr Ile Leu Lys Pro Asn Asp Ala Ile Asn Phe Glu Ser
   260 265 270
   Asn Gly Asn Phe Ile Ala Pro Glu Tyr Ala Tyr Lys Ile Val Lys Lys
   275 280 285
   Gly Asp Ser Ala Ile Met Lys Ser Glu Leu Glu Tyr Gly Asn Cys Asn
   290 295 300
   Thr Lys Cys Gln Thr Pro Met Gly Ala Ile Asn Ser Ser Met Pro Phe
   305 310 315 320
   His Asn Ile His Pro Leu Thr Ile Gly Glu Cys Pro Lys Tyr Val Lys
   325 330 335
   Ser Asn Arg Leu Val Leu Ala Thr Gly Leu Arg Asn Ser Pro Gln Arg
   340 345 350
   Glu Ser Arg Arg Lys Lys Arg Gly Leu Phe Gly Ala Ile Ala Gly Phe
   355 360 365
   Ile Glu Gly Gly Trp Gln Gly Met Val Asp Gly Trp Tyr Gly Tyr His
   370 375 380
   His Ser Asn Glu Gln Gly Ser Gly Tyr Ala Ala Asp Lys Glu Ser Thr
   385 390 395 400
   Gln Lys Ala Ile Asp Gly Val Thr Asn Lys Val Asn Ser Ile Ile Asp
   405 410 415
   Lys Met Asn Thr Gln Phe Glu Ala Val Gly Arg Glu Phe Asn Asn Leu
               420                 425                 430
   Glu Arg Arg Ile Glu Asn Leu Asn Lys Lys Met Glu Asp Gly Phe Leu
           435                 440                 445
   Asp Val Trp Thr Tyr Asn Ala Glu Leu Leu Val Leu Met Glu Asn Glu
       450                 455                 460
   Arg Thr Leu Asp Phe His Asp Ser Asn Val Lys Asn Leu Tyr Asp Lys
   465                 470                 475                 480
   Val Arg Leu Gln Leu Arg Asp Asn Ala Lys Glu Leu Gly Asn Gly Cys
                   485                 490                 495
   Phe Glu Phe Tyr His Lys Cys Asp Asn Glu Cys Met Glu Ser Ile Arg
               500                 505                 510
   Asn Gly Thr Tyr Asn Tyr Pro Gln Tyr Ser Glu Glu Ala Arg Leu Lys
           515                 520                 525
   Arg Glu Glu Ile Ser Gly Val Lys Leu Glu Ser Ile Gly Thr Tyr Gln
       530                 535                 540
   Ile Leu Ser Ile Tyr Ser Thr Val Ala Ser Ser Leu Ala Leu Ala Ile
   545                 550                 555                 560
   Met Met Ala Gly Leu Ser Leu Trp Met Cys Ser Asn Gly Ser Leu Gln
                   565                 570                 575
   Cys Arg Ile Cys Ile
               580
<210> 2
   <211> 594
   <212> PRT
   <213> Avian influenza virus
<400> 2
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala Asp Gln
               20                  25                  30
   Ile Cys Ile Gly Tyr His Ala Asn Asn Ser Thr Glu Gln Val Asp Thr
           35                  40                  45
   Ile Met Glu Lys Asn Val Thr Val Thr His Ala Gln Asp Ile Leu Glu
       50                  55                  60
   Lys Thr His Asn Gly Lys Leu Cys Asp Leu Asp Gly Val Lys Pro Leu
   65                  70                  75                  80
   Ile Leu Arg Asp Cys Ser Val Ala Gly Trp Leu Leu Gly Asn Pro Met
                   85                  90                  95
   Cys Asp Glu Phe Ile Asn Val Pro Glu Trp Ser Tyr Ile Val Glu Lys
               100                 105                 110
   Ala Asn Pro Thr Asn Asp Leu Cys Tyr Pro Gly Ser Phe Asn Asp Tyr
           115                 120                 125
   Glu Glu Leu Lys His Leu Leu Ser Arg Ile Asn His Phe Glu Lys Ile
       130                 135                 140
   Leu Ile Ile Pro Lys Ser Ser Trp Ser Asp His Glu Ala Ser Ser Gly
   145                 150                 155                 160
   Val Ser Ser Ala Cys Pro Tyr Leu Gly Ser Pro Ser Phe Phe Arg Asn
                   165                 170                 175
   Val Val Trp Leu Ile Lys Lys Asn Ser Thr Tyr Pro Thr Ile Lys Lys
               180                 185                 190
   Ser Tyr Asn Asn Thr Asn Gln Glu Asp Leu Leu Val Leu Trp Gly Ile
           195                 200                 205
   His His Pro Asn Asp Ala Ala Glu Gln Thr Arg Leu Tyr Gln Asn Pro
       210                 215                 220
   Thr Thr Tyr Ile Ser Ile Gly Thr Ser Thr Leu Asn Gln Arg Leu Val
   225                 230                 235                 240
   Pro Lys Ile Ala Thr Arg Ser Lys Val Asn Gly Gln Ser Gly Arg Met
                   245                 250                 255
   Glu Phe Phe Trp Thr Ile Leu Lys Pro Asn Asp Ala Ile Asn Phe Glu
               260                 265                 270
   Ser Asn Gly Asn Phe Ile Ala Pro Glu Tyr Ala Tyr Lys Ile Val Lys
           275                 280                 285
   Lys Gly Asp Ser Ala Ile Met Lys Ser Glu Leu Glu Tyr Gly Asn Cys
       290                 295                 300
   Asn Thr Lys Cys Gln Thr Pro Met Gly Ala Ile Asn Ser Ser Met Pro
   305                 310                 315                 320
   Phe His Asn Ile His Pro Leu Thr Ile Gly Glu Cys Pro Lys Tyr Val
                   325                 330                 335
   Lys Ser Asn Arg Leu Val Leu Ala Thr Gly Leu Arg Asn Ser Pro Gln
               340                 345                 350
   Arg Glu Ser Arg Arg Lys Lys Arg Gly Leu Phe Gly Ala Ile Ala Gly
           355                 360                 365
   Phe Ile Glu Gly Gly Trp Gln Gly Met Val Asp Gly Trp Tyr Gly Tyr
       370                 375                 380
   His His Ser Asn Glu Gln Gly Ser Gly Tyr Ala Ala Asp Lys Glu Ser
   385                 390                 395                 400
   Thr Gln Lys Ala Ile Asp Gly Val Thr Asn Lys Val Asn Ser Ile Ile
                   405                 410                 415
   Asp Lys Met Asn Thr Gln Phe Glu Ala Val Gly Arg Glu Phe Asn Asn
               420                 425                 430
   Leu Glu Arg Arg Ile Glu Asn Leu Asn Lys Lys Met Glu Asp Gly Phe
           435                 440                 445
   Leu Asp Val Trp Thr Tyr Asn Ala Glu Leu Leu Val Leu Met Glu Asn
       450                 455                 460
   Glu Arg Thr Leu Asp Phe His Asp Ser Asn Val Lys Asn Leu Tyr Asp
   465                 470                 475                 480
   Lys Val Arg Leu Gln Leu Arg Asp Asn Ala Lys Glu Leu Gly Asn Gly
                   485                 490                 495
   Cys Phe Glu Phe Tyr His Lys Cys Asp Asn Glu Cys Met Glu Ser Ile
               500                 505                 510
   Arg Asn Gly Thr Tyr Asn Tyr Pro Gln Tyr Ser Glu Glu Ala Arg Leu
           515                 520                 525
   Lys Arg Glu Glu Ile Ser Gly Val Lys Leu Glu Ser Ile Gly Thr Tyr
       530                 535                 540
   Gln Ile Val Thr Leu Trp Ala Thr Val Phe Leu Thr Leu Gly Ala Leu
   545                 550                 555                 560
   Val Ala Gly Ala Lys Val Trp Glu Ile Met Arg Lys Ala Asn Arg Lys
   565 570 575
   Ser Gln Tyr Lys Arg Thr Asn Thr Glu Pro His Asp Ser Gln Ala Thr
   580 585 590
   Trp Ile
<210> 3
   <211> 176
   <212> PRT
   <213> Lettuce necrotic yellows virus
<400> 3
   Ser Ala Lys Leu Asn Trp Tyr Arg Ile Thr Phe Asn Asp Thr Val Trp
   1 5 10 15
   Arg Phe Asp Thr Ala Arg Gly Pro Lys Asp Gly Glu Thr Cys Pro Leu
   20 25 30
   Ile Ala Ser Glu Leu Phe Ser Ser Gly Leu Ser Glu Val Phe Lys Ser
   35 40 45
   Val Thr Ser Phe Ser Glu Ile Leu Arg Asn Met Glu Ser Arg Gly Tyr
   50 55 60
   Ile Thr Asn Ile Thr Leu Arg Ala Asp Ser Asp Ile Leu Gly Pro Gly
   65 70 75 80
   Ala Leu Arg Cys Glu Phe Leu Phe Pro Ser Glu Val Phe Ile Pro Thr
   85 90 95
   Ser His Thr Leu Lys Met Gly Arg Ser Ser Leu Ile Leu Glu Pro His
   100 105 110
   Leu Val Val Leu Lys Glu Cys Lys Tyr Ile Ser Ser Gly Lys Leu Asp
   115 120 125
   Ile Gly Ile Ser Ser Ile Glu Ala Thr Ser Val Ala Val Leu Arg Arg
   130 135 140
   Val Lys Gly Pro Ala Phe Ile Gly Cys Met Asp Asp Asn Pro Phe Gly
   145 150 155 160
   Val Leu Thr Lys Lys Pro Ser Asp Glu Lys Asn Val Leu Ala Ser Lys
   165 170 175
<210> 4
   <211> 252
   <212> PRT
   <213> Avian influenza virus
<400> 4
   Met Ser Leu Leu Thr Glu Val Glu Thr Tyr Val Leu Ser Ile Val Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Ala Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Asn Gly Asn Gly Asp Pro Asn Asn Met Asp Arg Ala
                   85                  90                  95
   Val Lys Leu Tyr Lys Lys Leu Lys Arg Glu Ile Thr Phe His Gly Ala
               100                 105                 110
   Lys Glu Val Ala Leu Ser Tyr Ser Thr Gly Ala Leu Ala Ser Cys Met
           115                 120                 125
   Gly Leu Ile Tyr Asn Arg Met Gly Thr Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Ala Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Gln
           195                 200                 205
   Ala Arg Gln Met Val Gln Ala Met Arg Thr Ile Gly Thr His Pro Ser
       210                 215                 220
   Ser Ser Ala Gly Leu Lys Asp Asn Leu Leu Glu Asn Leu Gln Ala Tyr
   225                 230                 235                 240
   Gln Lys Arg Met Gly Val Gln Met Gln Arg Phe Lys
                   245                 250

## Description

### FIELD OF THE INVENTION

The present invention relates generally to enveloped virus-like particles (VLPs) preferably produced in plants, and methods for making and using the VLPs.

### BACKGROUND OF THE INVENTION

Virus-like particles (VLPs) are complex structures formed by self-assembling viral proteins without the presence of the viral genome. The VLP platform is increasingly used to enhance target-specific immunogenicity. VLPs can be produced in several expression systems, including plants. Production of VLPs in plants offers additional advantages, including safety and time efficiency. However, formation of VLPs in plants is part of a physiological process and results in formation of particles of various sizes and shapes, making commercial manufacturing and a smooth regulatory path highly challenging. The size of wild-type influenza virus particles depends on the strain and varies between 70 and 270 nm (Nayak et al., 2009, Virus Res 143(2):147-161). Plant-produced influenza viral particles (VLPs) have different shapes and a broad size range. For example, enveloped pleiomorphic influenza VLPs have been reported to be 100 to 150 nm in size. (Vezina et al., 2011, BioPharm International Supplements 24(5):s27-s30). Thus, there remains a need for more uniform VLPs suitable vaccine development.

WO 2008/058396 A1 discloses VLPs comprising a coat protein of the non-enveloped papaya mosaic virus (PapMV) which present e.g. an influenza antigen.

WO 2011/0350004 A1 discloses VLPs comprising a plant viral coat protein fused to a target protein. In particular, VLPs comprising a coat protein of the non-enveloped virus alfafa mosaic virus (AIMV) fused to a hemagglutinin from influenza virus is disclosed.

### SUMMARY OF THE INVENTION

The disclosed subject matter of the present invention relates to novel virus-like particles (VLPs) having a matrix protein derived from a first plant rhabdovirus and a surface polypeptide and their uses. These VLPs are enveloped, and substantially uniform in size.

According to one aspect of the present invention, a virus-like particle (VLP) is provided. The VLP comprises a matrix protein from a first plant enveloped rhabdovirus and a surface polypeptide. The surface polypeptide comprises a surface exposed portion from a target polypeptide, wherein the target polypeptide is from an animal virus, bacterium, parasite or fungus and wherein the surface exposed portion of the surface polypeptide is antigenic, a transmembrane domain, and a cytosolic tail. The cytosolic tail is from a transmembrane protein (e.g., glycoprotein) of a second plant rhabdovirus. The VLP may be produced in a plant cell, a plant, or a portion of a plant.

The first and the second plant rhabdoviruses may be the same or different, preferably the same. The plant rhabdovirus may be selected from the group consisting of Lettuce Necrotic Yellows virus (LNYV), Northern Cereal Mosaic virus (NCMV), Sonhus Virus (SonV) and Broccoli necrotic yellows virus (BNYV). Preferably, the plant rhabdovirus is Lettuce Necrotic Yellows virus (LNYV).

The surface exposed portion of the surface polypeptide is an antigenic polypeptide, such as a vaccine component, or a therapeutic agent. The therapeutic agent may be a therapeutic polypeptide.

The target polypeptide is selected from the group consisting of an animal virus, bacterium, parasite or fungus. The animal virus may be selected from the group consisting of an influenza virus, a respiratory syncytial virus (RSV), a human immunodeficiency virus (HIV), a hepatitis B virus (HBV), a hepatitis C virus (HCV), a human papillomavirus (HPV), an Ebola virus, a Yellow fever virus, a rotovirus, and a vesicular stomatitis virus (VSV).

The target polypeptide may be a native surface polypeptide. The native surface polypeptide may be a hemagglutinin of an influenza virus.

The target polypeptide may be an artificial surface polypeptide. The artificial surface polypeptide may be protective antigen 83 (PA83) from *Bacillus anthracis,* Pfs25 from *Plasmodium falciparum* or other soluble protein or peptide.

The influenza virus may be selected from the group consisting of an Influenza A virus and an Influenza B virus. The influenza virus may be selected from the group consisting of Influenza A Indonesia 05/05 strain, Influenza A virus California /04/2009 (H1N1) strain, Influenza A /Victoria /3/75 (H3N2) strain and Influenza B Hong Kong/330/2001.

The target polypeptide may be a therapeutic agent. The therapeutic agent may be a therapeutic polypeptide.

The transmembrane domain may be native or foreign to the surface exposed portion of the surface polypeptide. The transmembrane domain may be derived from a cellular or viral transmembrane protein. The viral transmembrane domain may be derived from the transmembrane protein from which the cytosolic tail is derived.

According to another aspect of the present invention, a method of producing virus-like particles (VLPs) in a plant cell, a plant, or a portion of a plant is provided. The method comprises introducing one or more nucleic acid molecules into the plant cell, the plant, or the portion of a plant. The one or more nucleic acid molecules comprise a first nucleotide sequence encoding a matrix protein from a first plant rhabdovirus and a second nucleotide sequence encoding a surface polypeptide. The surface polypeptide comprises a surface exposed portion from a target polypeptide, wherein the target polypeptide is from an animal virus, bacterium, parasite or fungus and wherein the surface exposed portion of the surface polypeptide is antigenic, a transmembrane domain, and a cytosolic tail. The cytosolic tail is from a transmembrane protein (e.g., glycoprotein) of a second plant rhabdovirus. The first and second plant rhabdoviruses may be the same or different, preferably the same. The method further comprises maintaining the plant cell, the plant, or the portion of a plant under conditions permitting co-expression of the matrix protein and the surface polypeptide such that the VLPs are produced. The VLPs may be substantially uniform in size. The method may further comprise purifying the VLPs from the plant cell, the plant or the portion of the plant.

The one or more nucleic acid molecules may be introduced into the plant cell, the plant or the portion of a plant by infiltration, particle bombardment, or inoculation. The one or more nucleic acid molecules may be introduced into the plant or a portion thereof transiently or stably.

Various immunogenic compositions are provided. In some embodiments, the immunogenic composition comprises an effective amount of the VLPs of the present invention, and the VLPs are substantially uniform in size. In other embodiments, the immunogenic composition comprises an effective amount of the VLPs produced by the method of the present invention, and the VLPs are substantially uniform in size. The immunogenic composition may further comprise an adjuvant and/or an excipient.

A method of inducing an immune response to the target polypeptide in a subject is described herein. The method comprises administering to the subject an effective amount of the immunogenic composition of the present invention, wherein the VLPs are substantially uniform in size.

A method of inducing a protective immune response to a pathogen in a subject is also described herein. The method comprises administering to the subject an effective amount of the immunogenic composition of the present invention, wherein the VLPs are substantially uniform in size. The target polypeptide is from the pathogen. The pathogen may be an influenza virus. The target polypeptide may be derived from a hemagglutinin.

A recombinant plant cell comprising one or more nucleic acid molecules is further provided. The one or more nucleic acid molecules comprise a first nucleotide sequence encoding a matrix protein from a first plant rhabdovirus and a second nucleotide sequence encoding a surface polypeptide. The surface polypeptide comprises (a) a surface exposed portion from a target polypeptide, wherein the target polypeptide is from an animal virus, bacterium, parasite or fungus and wherein the surface exposed portion of the surface polypeptide is antigenic, (b) a transmembrane domain, and (c) a cytosolic tail from a transmembrane protein (e.g., glycoprotein) of a second plant rhabdovirus. The first and second rhabdoviruses may be the same or different, preferably the same. Also provided is a plant or a portion thereof comprising the plant cell of the present invention.

### BRIEF DESCRIPTION OF THE DRAWINGS

Figure 1 shows (A) an electron microscopy image and (B) the organization of a plant rhabdovirus.
Figure 2 is a diagram illustrating the genome organization of the T-DNA region of a miniBYV vector for co-expression of two target proteins, i.e., Target 1 and Target 2, each operatively linked to a regulatory region. L-Pro, papain like leader proteinase; Met, Hel and Pol, methyltransferase, RNA helicase, RNA-dependent RNA polymerase domains of the replicase, respectively; 2Enx35S, 35S promoter with dual enhancers from Cauliflower mosaic virus; NOS - *Nopaline synthase* terminator; LB and RB, left and right borders of the T-DNA, respectively.

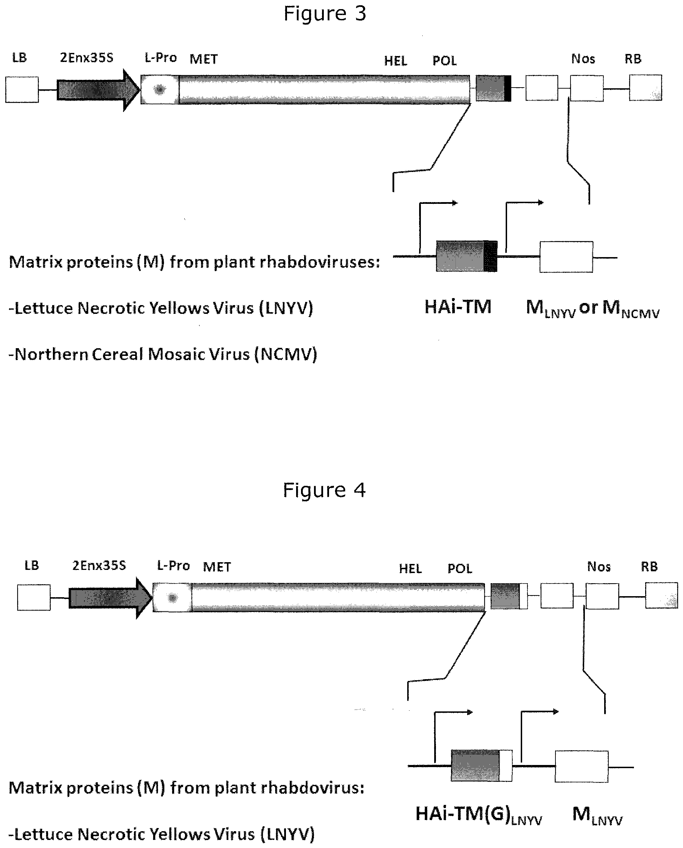

Figure 3 is a diagram illustrating the genome organization of the T-DNA region of a miniBYV-HAi-TM/M_{LNYV} or miniBYV-HAi-TM/M_{NCMV} vector for co-expression of an HAi-TM protein and a matrix protein of Lettuce Necrotic Yellows virus (LNYV) (M_{LNYV}) or Northern Cereal Mosaic virus (NCMV) (M_{NCMV}), respectively. L-Pro, papain like leader proteinase; Met, Hel and Pol, methyltransferase, RNA helicase, RNA-dependent RNA polymerase domains of the replicase, respectively; 2Enx35S; 35S promoter with dual enhancers from Cauliflower mosaic virus; NOS - *Nopaline synthase* terminator; LB and RB, left and right borders of the T-DNA, respectively.
Figure 4 is a diagram illustrating the genome organization of the T-DNA region of a miniBYV-HAi-TM(G)/M_{LNYV} vector for co-expression of an HAi-TM(G)_{LNYN} protein and an M_{LNYV} protein. L-Pro, papain like leader proteinase; Met, Hel and Pol, methyltransferase, RNA helicase, RNA-dependent RNA polymerase domains of the replicase, respectively; 2Enx35S, 35S promoter with dual enhancers from Cauliflower mosaic virus; NOS - *Nopaline synthase* terminator; LB and RB, left and right borders of the T-DNA, respectively.
Figure 5 depicts a representative western blot for time course analysis of HAi-TM expression detected by an anti-HAi 5G6 primary monoclonal antibody after infiltration. Reference HAi in the amount of 60, 30, or 15 ng was used as standard for quantification. Lanes 1 and 2, manual infiltrated *N. benthamiana;* 3 and 4, vacuum infiltrated N. *benthamiana.*
Figure 6 shows (A) distribution of HAi-TM VLPs through a 10%-40% sucrose gradient and (B) morphology of the HAi-TM VLPs from fraction 10 of (A) detected by transmission electron microscopy (TEM) negative staining.
Figure 7 shows (A) distribution of HAi-TM/M1 VLPs through a 10-40% sucrose gradient; and (B) morphology of the HAi-TM/M1 VLPs from fraction 10 of (A) detected by TEM negative staining.
Figure 8 shows (A) expression of the influenza A/Indonesia/05/2005 matrix (M1) protein in leaf samples (lanes 1-3, different replicas from the infiltrated plants producing HAi-TM/M1 VLPs); and (B) absence of the M1 protein from the HAi-TM/M1 VLPs in the 10-40% sucrose gradient fractions as shown on Figure 7A. Std, M1 protein standard.
Figure 9 shows (A) distribution HAi-TM/M_{LNYV} VLPs in a 10-40% sucrose gradient and (B) immunogold labeling of the HAi-TM/M_{LNYV} VLPs with an anti-HAi 5G6 mouse monoclonal antibody.
Figure 10 shows (A) distribution of HAi-TM(G)/M_{LNYV} VLPs through a 10%-40% sucrose gradient and (B) immunogold labeling of the HAi-TM(G)-M_{LNYV} VLPs from fraction 7 of (A) with an anti-HAi 5G6 mouse monoclonal antibody.
Figure 11 shows the amino acid sequences of (A) a hemagglutinin of H5N1 avian influenza virus (A/Indonesia/05/2005) (HAi-TM) (SEQ ID NO: 1) having a PR1a *Nicotiana tabacum* signal peptide (underlined), an HAi ectodomain, an HAi transmembrane domain (bold), and an HAi cytosolic tail (italicized), and (B) a hemagglutinin of H5N1 avian influenza virus (A/Indonesia/05/2005) (HAi-TM(G)_{LNYV}) (SEQ ID NO: 2) having a PR1a *N*. *tabacum* signal peptide (underlined), an HAi ectodomain , a transmembrane domain of an LNYV glycoprotein (bold), and a cytosolic tail of an LNYV glycoprotein (italicized).
Figure 12 shows the amino acid sequences of (A) a matrix protein of LNYV (M_{LNYV}) (SEQ ID NO: 3) and (B) a matrix protein of H5N1 avian influenza virus (A/Indonesia/05/2005) (M1 protein) (SEQ ID NO: 4).

### DETAILED DESCRIPTION OF THE INVENTION

The present invention is based on the discovery that novel virus-like particles (VLPs) comprising a matrix protein from a plant rhabdovirus and a surface polypeptide can be produced in plants. These VLPs are enveloped, and are substantially uniform in size, and may be used to design and manufacture effective human vaccines.

The term "protein" used herein refers to a biological molecule comprising amino acid residues. A protein may comprise one or more polypeptides. Each polypeptide may be a subunit of a protein. The protein may be in a native or modified form, and may exhibit a biological function when its polypeptide or polypeptides are properly folded or assembled.

The term "polypeptide," used herein refers to a polymer of amino acid residues with no limitation with respect to the minimum length of the polymer. Preferably, the polypeptide has at least 4 amino acids. A polypeptide may be a full-length protein, or a fragment or variant thereof.

The term "fragment" of a protein as used herein refers to a polypeptide having an amino acid sequence that is the same as a part, but not all, of the amino acid sequence of the protein. Preferably, a fragment is a functional fragment of a protein that retains the same function as the protein.

The term "variant" of a protein used herein refers to a polypeptide having an amino acid sequence that is the same as that of the protein except having at least one amino acid modified, for example, deleted, inserted, or replaced, respectively. The amino acid replacement may be a conservative amino acid substitution, preferably at a non-essential amino acid residue in the protein. A "conservative amino acid substitution" is one in which the amino acid residue is replaced with an amino acid residue having a similar side chain. Families of amino acid residues having similar side chains are known in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), non-polar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). A variant of a protein may have an amino acid sequence at least about 80%, 90%, 95%, or 99%, preferably at least about 90%, more preferably at least about 95%, identical to the amino acid sequence of the protein. Preferably, a variant is a functional variant of a protein that retains the same function as the protein.

The term "derived from" used herein refers to an origin or source, and may include naturally occurring, recombinant, unpurified or purified molecules. The molecules of the present invention may be derived from viral or non-viral molecules. A protein or polypeptide derived from an original protein or polypeptide may comprise the original protein or polypeptide, in part or in whole, and may be a fragment or variant of the original protein or polypeptide.

The term "native" used herein refers to a molecule (e.g., a protein or polypeptide) that is naturally occurring. The term "artificial" used herein refers to a molecule (e.g., a protein or polypeptide) that is not naturally occurring, but synthesized artificially, for example, recombinantly or chemically.

According to one aspect of the present invention, a virus-like particle (VLP) is provided comprising a matrix protein from a first plant rhabdovirus and a surface polypeptide, wherein the surface polypeptide comprises
(a) a surface exposed portion from a target polypeptide, wherein the target polypeptide is from an animal virus, bacterium, parasite or fungus and wherein the surface exposed portion of the surface polypeptide is antigenic
(b) a transmembrane domain, and
(c) a cytosolic tail from a transmembrane protein of a second plant rhabdovirus.

The VLP comprises a matrix protein and a surface polypeptide. The matrix protein is from a first plant rhabdovirus, more preferably, lettuce necrotic yellows virus. The surface polypeptide comprises a surface exposed portion from a target wherein the target polypeptide is from an animal virus, bacterium, parasite or fungus and wherein the surface exposed portion of the surface polypeptide is antigenic, a transmembrane domain and a cytosolic tail. The cytosolic tail is from a transmembrane protein (e.g., glycoprotein) of a second plant rhabdovirus. For example, the cytosolic tail may be derived from a rhabdoviral glycoprotein. The transmembrane domain and the cytosolic tail may be derived from the same or different plant enveloped viruses, preferably from the same plant enveloped virus. In one embodiment, the transmembrane domain is derived from a target protein while the cytosolic tail is from a rhabdovirus glycoprotein. In another embodiment, the transmembrane domain and the cytosolic tail are both derived from the same plant rhabdoviral glycoprotein. The matrix protein, the transmembrane domain and the cytosolic tail may be derived from the same plant rhabdovirus. The VLP may be produced in a plant cell or other cells. The plant cell may be a cell in a plant, a plant part (e.g., leaf, stem, root, floral tissue, seed or petiole), or a cell culture medium. The plant may be a whole growing plant. Preferably, the plant cell is in a plant leaf. The cell culture media may be any media suitable for growing plant cells, preferably in suspension. The plant cell is preferably suitable for expression of a VLP. For example, the plant cell may be in *N. benthamiana* leaves. Other suitable plants include *Nicotiana clevelandii, Beta vulgaris, Spinacia oleracea, Brassica* spp, *Lactuca sativa, Pisum sativum, Nicotiana tabacum, Plantago lanceolata, Tetragonia tetragonioides, Montia perfoliata, Stellaria media, Medicago truncatula,* and *Chenopodium foliosum.*

The VLP may comprise a membrane. The surface polypeptide of the VLP may be integrated into the membrane. The membrane may be derived from the cell in which the VLP is assembled, made or produced.

The matrix protein may be from a natural matrix protein of any plant rhabdovirus. The matrix protein may have an amino acid sequence at least about 80%, 85%, 90%, 95%, or 99%, preferably at least about 90%, more preferably at least about 95%, further preferably at least about 99%, most preferably about 100%, identical to that of the corresponding natural matrix protein.

The surface exposed portion derived from a target polypeptide is a portion of the surface polypeptide that is on the surface of the VLP. The surface polypeptide is an antigenic polypeptide, such as a vaccine component, or a therapeutic agent. The therapeutic agent may be a therapeutic polypeptide. The surface polypeptide, either in the VLP or purified from the VLP, may be used for various purposes. For example, the surface polypeptide is antigenic and used to induce an immune response in a subject when introduced into the subject. It may also be used as a therapeutic agent to treat a disease or disorder in a subject when administered to the subject. It may further be used as a diagnostic agent for diagnosis of a disease or disorder in a subject when used in testing a sample from the subject suspected of having the disease or disorder.

The surface polypeptide is from an antigenic target polypeptide, and capable of inducing an immune response in a subject when introduced into the subject, and may be used to form or become a vaccine candidate. It may comprise one or more epitopes (linear and/or conformational) capable of stimulating the immune system of a subject to make a humoral and/or cellular antigen-specific immune response. A humoral immune response refers to an immune response mediated by antibodies produced by B lymphocytes, or B cells, while a cellular immune response refers to an immune response mediated by T lymphocytes, or T cells, and/or other white blood cells. In general, a B-cell epitope contains at least about 5 amino acids but can be 3-4 amino acids, while a T-cell epitope includes at least about 7-9 amino acids and a helper T-cell epitope includes at least 12-20 amino acids. The target polypeptide may be derived from, in part or in whole, a natural protein (e.g., a surface protein or toxin subunit) of a pathogenic organism or pathogen. The target polypeptide may comprise an amino acid sequence at least about 10%, 20%, 30%, 40%, 50%, 60%, 70%,80%, 85%, 90%, 95%, or 99%, preferably at least about 90%, more preferably at least about 95%, further preferably at least about 99%, most preferably about 100%, identical to that of the corresponding natural pathogenic protein. The pathogen may be an intracellular or extracellular pathogen. The pathogen is selected from animal viruses, bacteria, parasites and fungi. An animal virus may be selected from the group consisting of an influenza virus, a respiratory syncytial virus (RSV), a human immunodeficiency virus (HIV), a hepatitis B virus (HBV), a hepatitis C virus (HCV), a human papillomavirus (HPV), an Ebola virus, a Yellow fever virus, a rotovarus, and a vesicular stomatitis virus (VSV). Preferably, the target polypeptide is derived from an influenza virus.

The target polypeptide may be a native surface polypeptide or an artificial surface polypeptide. The native surface polypeptide may be a hemagglutinin of an influenza virus. The artificial surface polypeptide may be protective antigen 83 (PA83), Pfs25, or other soluble protein or peptide. The PA83 is from *Bacilus anthracis.* The Pfs25 is from *Plasmodium falciparum.*

An influenza virus may be selected from the group consisting of an Influenza A virus and an Influenza B virus. The influenza virus may be selected from the group consisting of Influenza A Indonesia 05/05 strain, Influenza A virus California /04/2009 (H1N1) strain, Influenza A /Victoria /3/75 (H3N2) strain and Influenza B Hong Kong/330/2001.

The transmembrane domain of the surface polypeptide may be a native protein or an artificial protein. The transmembrane domain may be native or foreign to the surface exposed portion of the surface polypeptide or the target polypeptide. The transmembrane domain may be derived from a transmembrane protein, preferably the same transmembrane protein, from which the matrix protein and the cytosolic tail are derived. The transmembrane domain may be derived from a cellular or viral transmembrane protein, or a surface protein. The transmembrane domain may or may not be derived from the target polypeptide. The viral transmembrane protein may be a transmembrane protein from a plant virus. The plant virus may be a plant enveloped virus, preferably the plant enveloped virus from which the matrix protein and the cytosolic tail are derived. The transmembrane domain may be derived from a glycoprotein of a virus, preferably a glycoprotein of a plant enveloped virus, more preferably a glycoprotein of the plant enveloped virus from which the matrix protein and the cytosolic tail are derived.

A plant enveloped virus has a lipid envelope. This lipid envelope is typically derived from the membrane of a host cell from which the virus particle buds off. The plant enveloped virus may be a plant rhabdovirus or non-rhabdovirus. Examples of plant rhabdoviruses include Lettuce Necrotic Yellows virus (LNYV), Northern Cereal Mosaic virus (NCMV), Sonhus Virus (SonV) Broccoli necrotic yellows virus (BNYV), and other plant rhabdoviruses recognized by the International Committee on Taxonomy of Viruses (ICTV). Preferably, the plant enveloped virus is Lettuce Necrotic Yellows virus (LNYV).

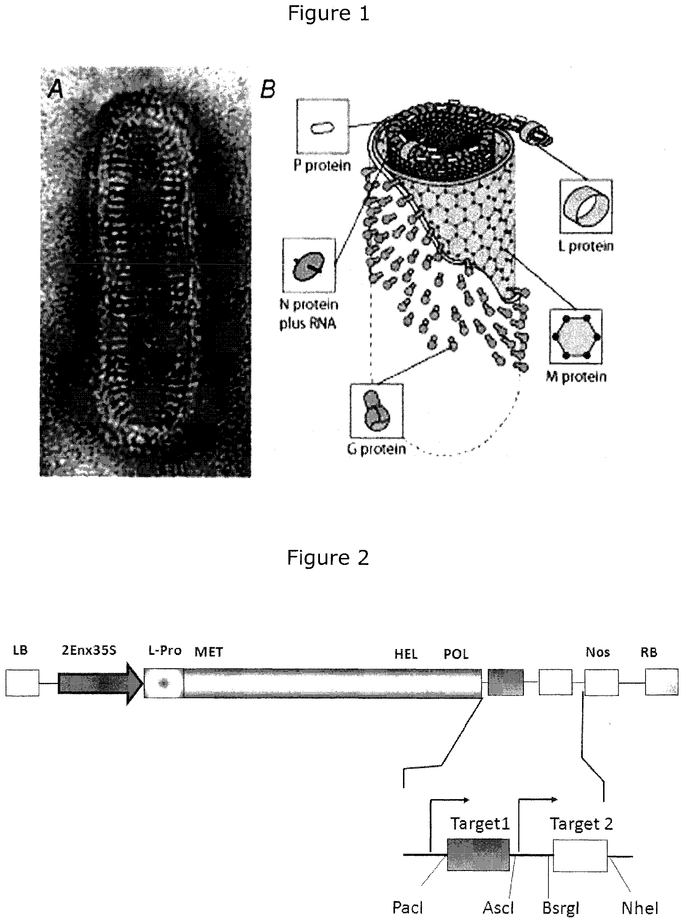

LNYV and NCMV rhabdoviruses are characterized by monocistronic negative sense RNA and belong to the family Rhabdoviridae and the genus Cytorhabdoviruses. Plant rhabdoviruses are characterized by the bacilli-form structure (Fig. 1A) and are about 130-350 nm in length and about 40-100 nm in width (Jackson et al, 2005, Ann. Rev. Phytopathol. 43:623-660). The two proteins of the virus that are key for assembly of bacilli-form particles are matrix protein (M protein) and glycoprotein (G protein). M protein forms a layer inside the virion's envelope and interacts with the cytosolic tale of G protein (Fig. 1B).

According to another aspect of the present invention, a method for producing the VLPs of the present invention in a plant cell, a plant, or a portion of a plant is provided. The method comprises introducing one or more nucleic acid molecules into the plant cell, the plant, or the portion of a plant. The one or more nucleic acid molecules comprise a first nucleotide sequence encoding the matrix protein and a second nucleotide sequence encoding the surface polypeptide. The matrix protein is from a first plant rhabdovirus. The surface polypeptide comprises a surface exposed portion derived from a target polypeptide, wherein the target polypeptide is from an animal virus, bacterium, parasite or fungus and wherein the surface exposed portion of the surface polypeptide is antigenic, a transmembrane domain, and a cytosolic tail. The cytosolic tail is from a transmembrane protein (e.g., glycoprotein) of a second plant rhabdovirus. For example, the cytosolic tail may be derived from a plant rhabdoviral glycoprotein. The transmembrane domain may or may not be derived from the target polypeptide. The first and the second plant rhabdoviruses may be the same or different, preferably the same. The method further comprises maintaining the plant cell, the plant, or the portion of a plant under conditions permitting co-expression of the matrix protein and the surface polypeptide such that the VLPs are produced.

The VLPs produced by a method according to the present invention may be of any sizes. For example, the VLPs may have a diameter in the range of about 5-350 nm, including about 40-60 nm. Preferably, the VLPs are substantially uniform in size. The term "substantially uniform in size" used herein means that at least about 50%, 60%, 70%, 80%, 90%, 95% or 99%, preferably at least about 80%, more preferably at least about 90%, most preferably at least about 95%, of the VLPs have a diameter within less than about 50%, 45%, 40%, 35%, 30%, 25%, 20%, 15%, 10%, or 5%, preferably less than about 30%, more preferably less than about 20%, most preferably less than 10%, of the average diameter of all the VLPs. The diameter of a VLP may be the average or mean value of one or more measurements of the diameter of the VLP. The diameter of a VLP may be determined by conventional techniques known in the art, for example, by observation using an electron microscopy, sucrose gradient fractionation, size exclusion chromatography, and dynamic light scattering. The diameter values of the VLPs may be analyzed using various software, for example, ImageJ software (NIH, Bethesda, MD) and other commercially available software.

The one or more nucleic acid molecules may further comprise a regulatory region operatively linked to the first or second nucleotide sequence. The regulatory region may be activated in a plant cell for the expression of the protein encoded by the first or the second nucleotide sequence. The regulatory region may include a promoter, for example, a plant viral promoter. Preferably, the first and second nucleotide sequences are in the same nucleic acid molecule, but linked to separate regulatory regions. The one or more nucleic acid molecules may further comprise a third nucleotide sequence encoding an additional surface polypeptide.

For each nucleic acid molecule, a vector comprising the nucleotide sequence of the nucleic acid molecule is provided. The vector may include a border sequence of a bacterial transfer DNA at either end, situated in a bacterial transfer DNA, to allow for delivery of the nucleic acid into a plant cell. Specifically, the vector may comprise one or more nucleic acid sequences derived from a Ti plasmid of a binary vector, and may also comprise a plant viral sequence. Such a vector, including elements of a Ti plasmid and a viral vector, is also called a launch vector. This vector may also be used for co-expression of the protein or polypeptide of interest (e.g., the matrix protein and/or the surface polypeptide) with a protein such as a silencing suppressor or a post-translational modifying enzyme such as PNGaseF, to modify, affect expression and/or increase production of the protein of interest by, for example, facilitating maturation or accumulation of the protein.

Each nucleic acid molecule may be introduced into the plant cell, the plant, or the portion of a plant (e.g., leaf, stem, root, floral tissue, seed or petiole) using techniques known in the art. For example, the nucleic acid molecule may be delivered via infiltration, particle bombardment, or inoculation. The nucleic acid molecule could be used as a part of an inducible system activated by, for example, chemical, light or heat shock. Preferably, the nucleic acid molecule is introduced into the plant cell via infiltration. The nucleic acid molecule may be introduced transiently or stably.

A recombinant plant cell comprising one or more nucleic acid molecules is provided. The one or more nucleic acid molecules comprise a first nucleotide sequence encoding a matrix protein and a second nucleotide sequence encoding a surface polypeptide. Preferably, the first and second nucleotide sequences are in one nucleic acid molecule. The matrix protein is from a first plant rhabdovirus. The surface polypeptide comprises a surface exposed portion from a target polypeptide, wherein the target polypeptide is from an animal virus, bacterium, parasite or fungus and wherein the surface exposed portion of the surface polypeptide is antigenic, a transmembrane domain and a cytosolic tail. The cytosolic tail is derived from a transmembrane protein (e.g., glycoprotein) of a second plant rhabdovirus The first and second plant rhabdoviruses may be the same or different, preferably the same. The transmembrane domain may or may not be derived from the target polypeptide. The cytosolic tail is from a glycoprotein of a plant rhabdovirus. For example, the cytosolic tail may be derived from a rhabdoviral glycoprotein. The first or second nucleotide sequence may be operatively linked to a promoter capable of being activated in the plant cell for co-expression of the matrix protein or the surface polypeptide, respectively. Also provided is a plant or a portion thereof comprising the plant cell of the present invention.

For production of the VLPs of the present invention, a plant cell, or a plant or a portion thereof may be maintained under conditions permitting co-expression of the matrix protein and the surface polypeptide, resulting in the production of the VLPs. Such conditions include suitable temperature, humidity, pressure, light/dark cycle, and illumination. Preferably, the matrix protein and the surface polypeptide are co-expressed in the same plant cells.

The production method may further comprise purifying the VLPs. Conventional purification techniques known in the art may be used. For example, the VLP may be purified from the plant cell using an antibody or a receptor capable of binding the surface polypeptide. The purification process may comprise extraction of the VLPs from the plant cell using an extraction buffer. After low speed centrifugation, supernatant may be clarified by filtration and used for chromatography. The purified VLPs may be at least about 10%, 20%, 30%, 40%, 50%, 60%, 70%, 80%, 90%, 95% or 99%, preferably at least about 50%, more preferably at least about 75%, most preferably at least about 95%, pure. The VLPs in a crude plant extract may be used.

An immunogenic composition is provided. The composition comprises an effective amount of the VLPs of the present invention. Preferably, the VLPs are substantially uniform in size. The effective amount of the VLPs is an amount of the VLPs sufficient to make the composition immunogenic, i.e., capable of inducing an immune response in a subject when introduced into the subject. The composition may further comprise an adjuvant and/or an excipient. The immunogenic compositions may be used to induce an immune response in a subject. Several methods of inducing an immune response are provided.

A method of inducing an immune response to the target polypeptide in a subject is described herein. The method comprises administering to the subject an effective amount of the immunogenic composition of the present invention. Preferably, the VLPs are substantially uniform in size. The immunogenic composition may be administered in an appropriate number of doses.

A method of inducing a protective immune response to a pathogen in a subject is also described herein. The method comprises administering to the subject an effective amount of the immunogenic composition of the present invention. Preferably, the VLPs are substantially uniform in size. The target polypeptide is derived from the pathogen. For example, the target polypeptide may be a hemagglutinin and the pathogen is an influenza virus.

The subject may be an animal, including a mammal, for example, a human, a mouse, a cow, a horse, a chicken, a dog, a cat, and a rabbit. The animal may be an agricultural animal (e.g., horse, cow and chicken) or a pet (e.g., dog and cat). The subject is preferably a human or a mouse, more preferably a human. The subject may be a male or female. The subject may also be a newborn, a child or an adult. The subject may have suffered or be predisposed to a disease or medical condition, which may be caused by or associated with a pathogen.

The immunogenic composition may be formulated, for example, for oral, sublingual, intranasal, intraocular, rectal, transdermal, mucosal, topical or parenteral administration. Parenteral administration may include intradermal, subcutaneous (s.c., s.q., sub-Q, Hypo), intranasal, intramuscular (i.m.), intravenous (i.v.), intraperitoneal (i.p.), intra-arterial, intramedulary, intracardiac, intra-articular (joint), intrasynovial (joint fluid area), intracranial, intraspinal, and intrathecal (spinal fluids) administration. Any device suitable for parenteral injection or infusion of the composition may be used for such administration.

The term "an effective amount" refers to an amount sufficient to achieve a stated goal, and may vary depending on the stated goal and other factors. For example, an effective amount of VLPs in an immunogenic composition or an effective amount of an immunogenic composition comprising VLPs may vary depending on the physical characteristics of the subject, the nature and severity of the need of the VLPs, the existence of related or unrelated medical conditions, the nature of the VLPs, the means of administering the VLPs to the subject, and the administration route. A specific dose for a given subject may generally be set by the judgment of a physician. The immunogenic composition may be administered to the subject in one or multiple doses.

Various medicaments comprising the VLPs of the present invention are described wherein. The VLPs are preferably substantially uniform in size. The medicaments may comprise suitable adjuvants. In some examples the medicaments are useful for inducing an immune response to the target polypeptide in a subject, and comprise an effective amount of the VLPs of the present invention. In some other examples, the medicaments are useful for inducing a protective immune response to a pathogen in a subject, and comprise an effective amount of the VLPs of the present invention, wherein the target polypeptide is derived from the pathogen. For example, the target polypeptide may be a hemagglutinin and the pathogen is an influenza virus.

For each medicament described herein, a method for preparing the medicament is disclosed. The preparation method comprises admixing the VLPs of the present invention with a pharmaceutically acceptable carrier and/or excipient. The VLPs are preferably substantially uniform in size.

The term "about" as used herein when referring to a measurable value such as an amount, a percentage, and the like, is meant to encompass variations of ±20% or ±10%, more preferably ±5%, even more preferably ±1%, and still more preferably ±0.1% from the specified value, as such variations are appropriate.

### Example 1. Construction of mini-BYV vectors

To demonstrate the feasibility of assembling VLPs using matrix proteins (M proteins) from different plant enveloped viruses, a rhabdoviral M protein and a target antigen containing a transmembrane (TM) domain and a cytosolic tail of a LNYV glycoprotein (G protein) were engineered for co-expression in plant cells. For co-expression of these proteins, a miniBYV vector was used (Fig. 2). The miniBYV vector contains a minireplicon derived from a *Closteroveridae* virus (e.g., *Beet yellows* virus), which comprises a nucleic acid sequence encoding only proteins required for replication of the *Closteroveridae* virus. Specifically, the M protein from LNYV or NCMV was co-expressed with a hemagglutinin (HAi) from H5N1 avian influenza virus (A/Indonesia/05/2005).

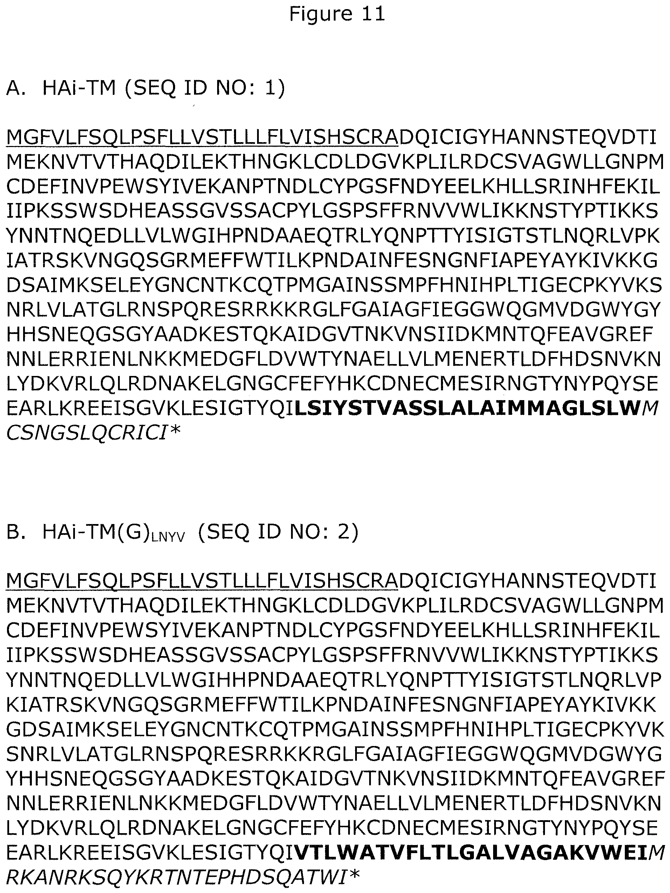

In brief, a gene encoding a HAi protein having its native TM domain (HAi-TM) was cloned into a miniBYV vector to generate the miniBYV-HAi-TM plasmid. The HAi-TM protein (SEQ ID NO: 1) included a PR1a *Nicotiana tabacum* signal peptide (underlined), an HAi ectodomain, an HAi native transmembrane domain (bold), and an HAi native cytosolic tail (italicized) (Fig. 11A). Subsequently, a gene encoding the M protein of LNYV (M_{LNYV} protein) or NCMV (M_{NCMV} protein) was introduced into the miniBYV-HAi-TM plasmid under the control of the closteroviral heterologous coat protein (CP) promoter to generate a miniBYV-HAi-TM/M_{LNVV} or miniBYV-HAi-TM/M_{NCMV} plasmid (Fig. 3). For VLP evaluation, positive clones were transformed into *Agrobacterium tumefaciens* strain GV3101 and then introduced into *Nicotiana benthamiana* plants to produce VLPs.

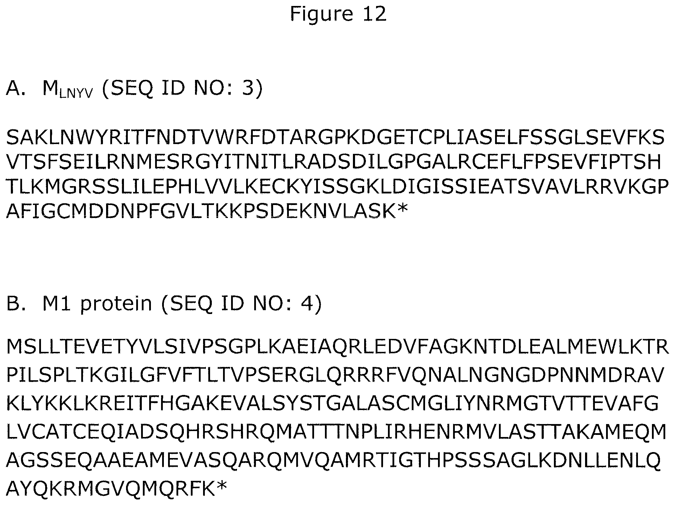

In parallel, the HAi gene was modified to encode a recombinant a hemagglutinin protein (HAi-TM(G)), in which the native TM and the cytosolic tail of HAi were replaced with the transmembrane domain and cytosolic tail from a glycoprotein of LNYV, respectively. A nucleotide sequence encoding the HAi-TM(G) protein (SEQ ID NO: 2), including a PR1a *Nicotiana tabacum* signal peptide (underlined), an HAi ectodomain, an LNYV glycoprotein transmembrane domain (bold), and an LNYV glycoprotein cytosolic tail (italicized) (Fig. 11B), was introduced into the miniBYV vector containing a nucleotide sequence encoding the M protein of LNYV (M_{LNYV} protein) (SEQ ID NO: 3) (Fig. 12A). The resulting construct, miniBYV-HAi-TM(G)/M_{LNYV} (Fig. 4), was sequenced, and positive clones were transformed into *A. tumefaciens* and then introduced into *Nicotiana benthamiana* plants to produce HAi-TM(G)-M_{LNYV} VLPs.

As a control, a gene encoding the matrix protein (M1 protein) of H5N1 avian influenza virus (A/Indonesia/05/2005) (SEQ ID NO: 4) (Fig. 12B) was introduced into the minBYV-HAi-TM plasmid under the control of the closteroviral heterologous coat protein (CP) promoter to generate the plasmid miniBYV-HAi-TM/M1. As described above, PR1a was used as a signal peptide. Positive clones were transformed into *A. tumefaciens* and then introduced into *Nicotiana benthamiana* plants to produce HAi-TM/M1 VLPs.

### Example 2. Optimization of infiltration procedure to express HAi

To evaluate the expression level of HAi and develop vacuum infiltration conditions, hydroponically grown *N. benthamiana* plants were used. To analyze the expression of HAi-TM, manual infiltration and vacuum infiltration of miniBYV-HAi-TM were performed using the same buffer. Five week old *Nicotiana benthamiana* plants grown in Rockwool in clam shells were used for vacuum and manual infiltration. Agrobacteria were grown in LB media, which was supplemented with 50 ng/ml Kanamycin and 50 ng/ml Hygromycin. One liter overnight cultures were grown at 28°C, shaking at 220 rpm for 18-24 hours.

Overnight concentration was determined by measuring the OD₆₀₀ for each culture. The agrobacteria were pelleted by spinning at 4000g for 15 min at 4°C. Bacterial pellets were re-suspended in 100 ml of fresh MMA media (10 mM MgCl2; 10 mM MES, pH 5.85; and 20 µM acetosyringone). The final concentration for each culture was recorded after rocking for 2 hours at room temperature. Cultures containing miniBYV and P1/HcPro silencing suppressor (miniBYV requires use of a silencing suppressor to achieve good target expression levels) were mixed to ratios **of** OD₆₀₀, 1.0:0.2, respectively. For vacuum infiltration, clamshells were infiltrated with miniBYV: P1/HcPro containing agrobacteria. Infiltrated plants were fed 50 ppm hydrosol and placed in a growth room for 5-8 days.

For manual infiltration, 3-5 leaves of hydroponically grown *Nicotiana benthamiana* were manually infiltrated using a 10cc syringe without a needle. Plants were fed with 50 ppm hydrosol and kept in the post infiltration room for 5-8 days.

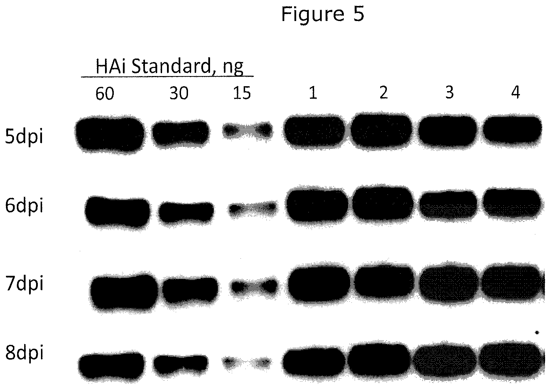

HAi-TM expression levels were determined based on the amount of HAi-TM protein detected by an anti-HAi 5G6 monoclonal antibody in Western blot analysis (Fig. 5). HAi-TM expression was higher in vacuum infiltrated leaves at 6 days post infiltrations (dpi) and 7 dpi compared to manually infiltrated leaves (Table 1). For example, at 6 dpi, the HAi-TM protein was expressed at 249 mg/kg in vacuum infiltrated leaves, representing a 43% increase in expression compared to manually infiltrated leaves.

**Table 1. Time course expression levels for HAi-TM**

| Days Post Infiltration | Manual Infiltration (mg/kg) | Vacuum Infiltration (mg/kg) |
|---|---|---|
| 5 | 73 | 46 |
| 6 | 174 | 249 |
| 7 | 120 | 123 |
| 8 | 180 | 105 |

### Example 3. Plant-produced HAi-TM VLPs

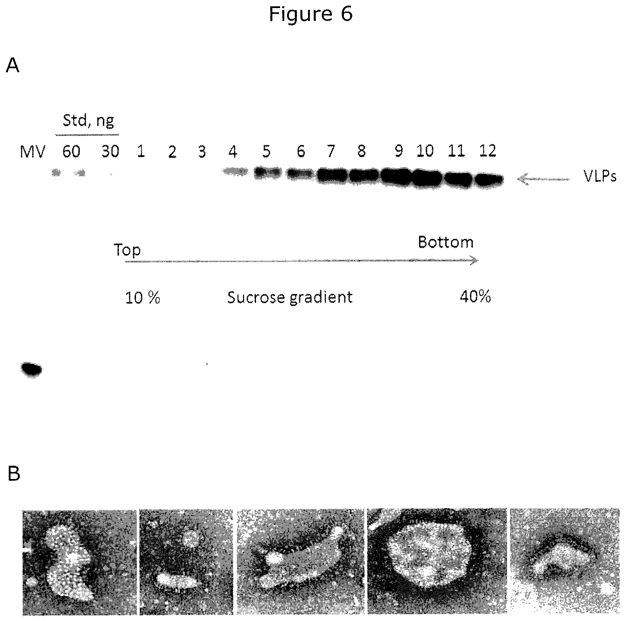

To characterize VLPs formation HAi-TM were cloned in to miniBYV vector using PacI/NheI restriction sites. The resulting plasmid miniBYV HAi-TM was transformed in to GV3101 Agrobacterium strain. *Nicothiana benthamiana* infiltrated leaves were harvested at 7 days post infiltration and VLPs were purified. Distribution of HAi-TM VLPs without any matrix protein through a 10%-40% sucrose gradient with peak fractions 9 and 10 was detected by an anti-HAi 5G6 mouse monoclonal antibody (developed by the Immunology group at Fraunhofer - USA Center for Molecular Biotechnology) (Fig. 6A). To evaluate the morphology of HAi-VLPs in fraction 10, VLPs from this fraction were analyzed by negative staining using transmission electron microscopy (TEM). HAi-TM VLPs with different sizes and shapes were observed (Fig. 6B).

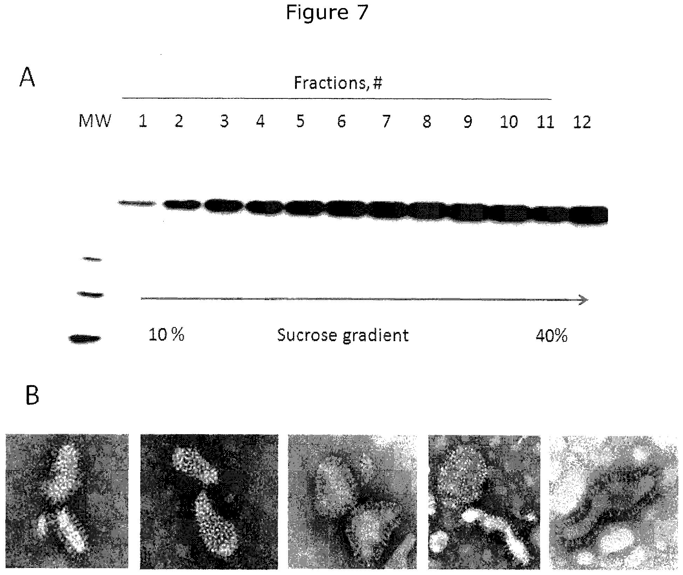

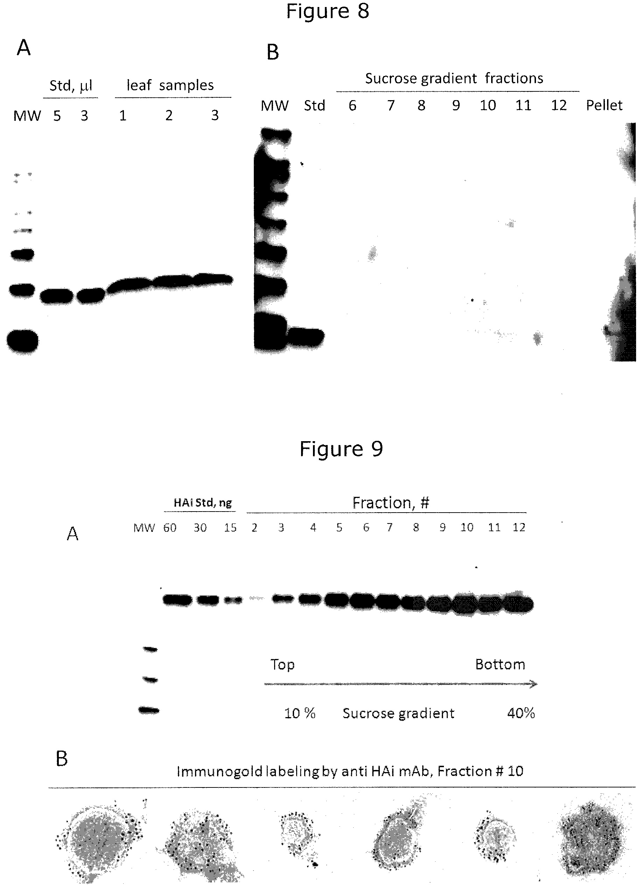

HAi-TM/M1 VLPs having HAi protein with its native transmembrane domain and cytosolic tail coexpressed with M1 of influenza A/Indonesia/05/05 were produced by expressing miniBYV-HAi-TM/M1 in plants using vacuum infiltration as described in Example 2. HAi-TM/M1 VLPs were purified using a sucrose gradient, and characterized by Western blot analysis and electron microscopy using the anti-HAi 5G6 mouse monoclonal antibody, and were found to distribute through a 10%-40% sucrose gradient with peak fractions 9 and 10 (Fig. 7A). No M1 protein incorporation was detected in the same HAi-TM/M1 VLPs through the 10-40% sucrose gradient (Fig. 8A) using a polyclonal goat anti-M1 antibody from Meredian Life science, Inc, USA. Meanwhile, the expression of M1 protein was confirmed by western blot using a goat anti-M1 protein polyclonal antibody (Fig. 8B). No uniformed VLPs were detected after co-expression HAi-TM and Influenza matrix M1 protein (Fig 7B).

### Example 4. Plant-produced HAi-TM/M_{LNYV} VLPs

HAi-TM/M_{LNYV} VLPs having HAi-TM protein (i.e., HAi protein with its native transmembrane domain and cytosolic tail) and LNYV M protein (M_{LNYV} protein) were produced by expressing miniBYV-HAi-TM/M_{LNYV} in plants using vacuum infiltration as described in Example 2. HAi-TM/M_{LNYV} VLPs were purified using a 10%-40% sucrose gradient and characterized by SDS-PAGE, Western blot analysis and electron microscopy. The presence of the HAi immunological determinant on the surface of these VLPs was confirmed by immunogold labeling using the anti-HAi 5G6 mouse monoclonal antibody (Fig. 9B). Also, this antibody was used to detect VLPs distribution in a 10-40% sucrose gradient with a peak in fraction 10 (Fig. 9A). The distribution of HAi-TM/M_{LNYV} VLPs was similar to that observed for HAi-TM/M1 VLPs (Figs. 7A and 9A). There was no noticeable difference in the VLP morphology (e.g., shape and size) (Figs. 7B and 9B).

### Example 5. Plant-produced uniform HAi-TM(G)/M_{LNYV} VLPs

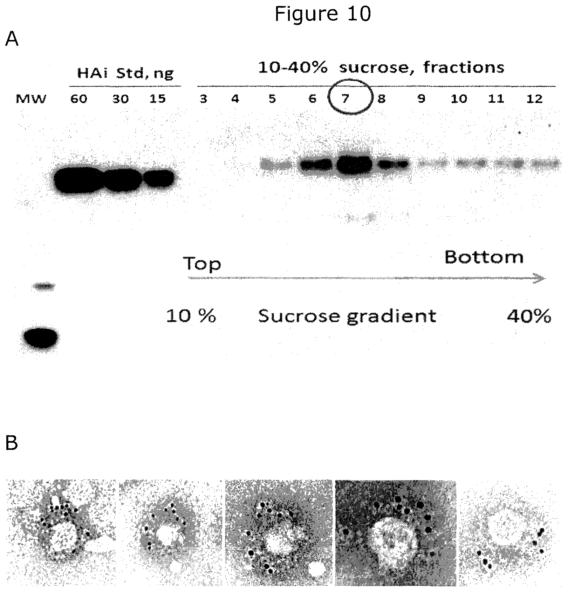

HAi-TM(G)/M_{LNYV} VLPs having HAi-TM(G) protein (i.e., HAi protein with a transmembrane domain and a cytosolic tail from a rhabdoviral glycoprotein (G) protein) and LNYV M protein (M_{LNYV} protein) were produced by expressing miniBYV-HAi-TM(G)/M_{LNYV} in plants using vacuum infiltration as described in Example 2. After separation of HAi-TM(G)/M_{LNYV} VLPs by a 10%-40% sucrose gradient, 1 mL fractions were collected and analyzed by Western blot analysis using the anti-HAi 5G6 monoclonal antibody. A shift in the target collection peak was observed. Unlike HAi-TM/M_{LNYV} VLPs, the HAi-TM(G)/M_{LNYV} VLPs were detected in fractions 6, 7 and 8 with a sharp peak in fraction 7 (Fig. 10A). Immunogold labeling following TEM showed round-shaped HAi-TM(G)/M_{LNYV} VLPs (Fig. 10B). The size of the HAi-TM(G)/M_{LNYV} VLPs determined by ImageJ software (NIH, Bethesda, MD) was 50.5±15 nm. The HAi-TM(G)-M_{LNYV} VLPs were substantially uniform in size and shape. Other embodiments of the invention will be apparent to those skilled in the art from consideration of the specification and practice of the invention disclosed herein. It is intended that the specification and examples be considered as exemplary only, with the true scope and spirit of the invention being indicated by the following claims.

## Claims

1. A virus-like particle comprising a matrix protein from a first plant rhabdovirus and a surface polypeptide, wherein the surface polypeptide comprises
(a) a surface exposed portion from a target polypeptide, wherein the target polypeptide is from an animal virus, bacterium, parasite or fungus and wherein the surface exposed portion of the surface polypeptide is antigenic
(b) a transmembrane domain, and
(c) a cytosolic tail from a transmembrane protein of a second plant rhabdovirus.

2. The virus-like particle of claim 1, wherein the first plant rhabdovirus and the second plant rhabdovirus are the same.

3. The virus-like particle of claim 1, wherein the plant rhabdovirus is selected from the group consisting of Lettuce Necrotic Yellows virus (LNYV), Northern Cereal Mosaic virus (NCMV), Sonhus Virus (SonV), and Broccoli necrotic yellows virus (BNYV).

4. A method of producing virus-like particles in a plant cell, a plant or a portion of a plant, comprising
(a) introducing one or more nucleic acid molecules into the plant cell, the plant or the portion of a plant, wherein the one or more nucleic acid molecules comprise a first nucleotide sequence encoding a matrix protein from a first plant rhabdovirus and a second nucleotide sequence encoding a surface polypeptide, wherein the surface polypeptide comprises
(i) a surface exposed portion from a target polypeptide, wherein the target polypeptide is from an animal virus, bacterium, parasite or fungus and wherein the surface exposed portion of the surface polypeptide is antigenic
(ii) a transmembrane domain, and
(iii) a cytosolic tail derived from a transmembrane protein of a second plant rhabdovirus; and
(b) maintaining the plant cell, the plant or the portion of a plant under conditions permitting co-expression of the matrix protein and the surface polypeptide, whereby the virus-like particles are produced.

5. The method of claim 4, wherein the first plant rhabdovirus and the second plant rhabdovirus are the same.

6. The method of claim 4, wherein at least 50% of the virus-like particles have a diameter within less than 50% of the average diameter of all the virus-like particles.

7. The method of claim 4, further comprising (c) purifying the virus-like particles.

8. An immunogenic composition comprising an effective amount of the virus-like particles of claim 1, wherein at least 50% of the virus-like particles have a diameter within less than 50% of the average diameter of all the virus-like particles.

9. An immunogenic composition comprising an effective amount of the virus-like particles produced by the method of claim 4, wherein at least 50% of the virus-like particles have a diameter within less than 50% of the average diameter of all the virus-like particles.

10. The virus-like particle of claim 1 or the virus-like particle produced by the method of claim 4 for use as a medicament, preferably for inducing a protective immune response to a pathogen in a subject.

11. A recombinant plant cell comprising one or more nucleic acid molecules, wherein the one or more nucleic acid molecules comprise a first nucleotide sequence encoding a matrix protein from a first rhabdovirus and a second nucleotide sequence encoding a surface polypeptide, and wherein the surface polypeptide comprises
(a) a surface exposed portion from a target polypeptide, wherein the target polypeptide is from an animal virus, bacterium, parasite or fungus and wherein the surface exposed portion of the surface polypeptide is antigenic
(b) a transmembrane domain, and
(c) a cytosolic tail from a glycoprotein of a second plant rhabdovirus.

12. The recombinant plant cell of claim 12, wherein the first plant rhabdovirus and the second plant rhabdovirus are the same.

13. A plant or a portion thereof comprising the plant cell of claim 11 or 12.

## Patentansprüche

1. Virusartiger Partikel, der ein Matrixprotein von einem ersten pflanzlichen Rhabdovirus und ein Oberflächenpolypeptid beinhaltet, wobei das Oberflächenpolypeptid Folgendes beinhaltet:
(a) einen oberflächenexponierten Teil von einem Targetpolypeptid, wobei das Targetpolypeptid von einem tierischen Virus, Bakterium, Parasiten oder Pilz stammt und wobei der oberflächenexponierte Teil des Oberflächenpolypeptids antigen ist,
(b) eine Transmembrandomäne und
(c) einen zytosolischen Schwanz von einem Transmembranprotein eines zweiten pflanzlichen Rhabdovirus.

2. Virusartiger Partikel nach Anspruch 1, wobei das erste pflanzliche Rhabdovirus und das zweite pflanzliche Rhabdovirus gleich sind.

3. Virusartiger Partikel nach Anspruch 1, wobei das pflanzliche Rhabdovirus ausgewählt ist aus der Gruppe bestehend aus Lettuce Necrotic Yellows-Virus (LNYV), Northern Cereal Mosaic-Virus (NCMV), Sonchus-Virus (SonV) und Broccoli Necrotic Yellows-Virus (BNYV).

4. Verfahren zum Produzieren virusartiger Partikel in einer Pflanzenzelle, einer Pflanze oder einem Teil einer Pflanze, das Folgendes beinhaltet:
(a) Einbringen von einem oder mehreren Nucleinsäuremolekülen in die Pflanzenzelle, die Pflanze oder den Teil einer Pflanze, wobei das eine oder die mehreren Nucleinsäuremolekül(e) eine erste Nucleotidsequenz, die ein Matrixprotein von einem ersten pflanzlichen Rhabdovirus kodiert, und eine zweite Nucleotidsequenz beinhaltet/beinhalten, die ein Oberflächenpolypeptid kodiert, wobei das Oberflächenpolypeptid Folgendes beinhaltet:
(i) einen oberflächenexponierten Teil von einem Targetpolypeptid, wobei das Targetpolypeptid von einem tierischen Virus, Bakterium, Parasiten oder Pilz stammt und wobei der oberflächenexponierte Teil des Oberflächenpolypeptids antigen ist,
(ii) eine Transmembrandomäne und
(iii) einen zytosolischen Schwanz, der von einem Transmembranprotein eines zweiten pflanzlichen Rhabdovirus abgeleitet ist; und
(b) Halten der Pflanzenzelle, der Pflanze oder des Teils einer Pflanze unter Bedingungen, die eine Coexpression des Matrixproteins und des Oberflächenpolypeptids zulassen, wodurch die virusartigen Partikel produziert werden.

5. Verfahren nach Anspruch 4, wobei das erste pflanzliche Rhabdovirus und das zweite pflanzliche Rhabdovirus gleich sind.

6. Verfahren nach Anspruch 4, wobei wenigstens 50 % der virusartigen Partikel einen Durchmesser innerhalb von weniger als 50 % des durchschnittlichen Durchmessers aller virusartigen Partikel haben.

7. Verfahren nach Anspruch 4, das ferner Folgendes beinhaltet:
(c) Reinigen der virusartigen Partikel.

8. Immunogene Zusammensetzung, die eine wirksame Menge der virusartigen Partikel nach Anspruch 1 beinhaltet, wobei wenigstens 50 % der virusartigen Partikel einen Durchmesser innerhalb von weniger als 50 % des durchschnittlichen Durchmessers aller virusartigen Partikel haben.

9. Immunogene Zusammensetzung, die eine wirksame Menge der virusartigen Partikel beinhaltet, die mit dem Verfahren nach Anspruch 4 produziert wurden, wobei wenigstens 50 % der virusartigen Partikel einen Durchmesser innerhalb von weniger als 50 % des durchschnittlichen Durchmessers aller virusartigen Partikel haben.

10. Virusartiger Partikel nach Anspruch 1 oder virusartiger Partikel, der mit dem Verfahren nach Anspruch 4 produziert wurde, zur Verwendung als Medikament, vorzugsweise zum Induzieren einer schützenden Immunreaktion auf ein Pathogen in einem Subjekt.

11. Rekombinante Pflanzenzelle, die ein oder mehrere Nucleinsäuremoleküle beinhaltet, wobei das eine oder die mehreren Nucleinsäuremolekül(e) eine erste Nucleotidsequenz, die ein Matrixprotein von einem ersten Rhabdovirus kodiert, und eine zweite Nucleotidsequenz beinhaltet/beinhalten, die ein Oberflächenpolypeptid kodiert, und wobei das Oberflächenpolypeptid Folgendes beinhaltet:
(a) einen oberflächenexponierten Teil von einem Targetpolypeptid, wobei das Targetpolypeptid von einem tierischen Virus, Bakterium, Parasiten oder Pilz stammt und wobei der oberflächenexponierte Teil des Oberflächenpolypeptids antigen ist,
(b) eine Transmembrandomäne und
(c) einen zytosolischen Schwanz von einem Glykoprotein eines zweiten pflanzlichen Rhabdovirus.

12. Rekombinante Pflanzenzelle nach Anspruch 12, wobei das erste pflanzliche Rhabdovirus und das zweite pflanzliche Rhabdovirus gleich sind.

13. Pflanze oder ein Teil davon, die/der die Pflanzenzelle nach Anspruch 11 oder 12 beinhaltet.

## Revendications

1. Pseudo-particule virale comprenant une protéine matricielle d'un premier rhabdovirus végétal et un polypeptide de surface, où le polypeptide de surface comprend
(a) une partie exposée en surface d'un polypeptide cible, où le polypeptide cible provient d'un virus animal, d'une bactérie, d'un parasite ou d'un mycète et où la partie exposée en surface du polypeptide de surface est antigénique
(b) un domaine transmembranaire et
(c) une queue cytosolique d'une protéine transmembranaire d'un deuxième rhabdovirus végétal.

2. Pseudo-particule virale de la revendication 1, où le premier rhabdovirus végétal et le deuxième rhabdovirus végétal sont les mêmes.

3. Pseudo-particule virale de la revendication 1, où le rhabdovirus végétal est sélectionné dans le groupe consistant en les suivants : virus de la jaunisse nécrotique de la laitue (LNYV), virus de la mosaïque des céréales du Nord (NCMV), sonchus virus (SonV) et virus de la jaunisse nécrotique du brocoli (BNYV).

4. Méthode de production de pseudo-particules virales dans une cellule végétale, une plante ou une partie d'une plante, qui comprend
(a) l'introduction de une ou plusieurs molécules d'acide nucléique dans la cellule végétale, la plante ou la partie d'une plante, où les une ou plusieurs molécules d'acide nucléique comprennent une première séquence nucléotidique codant pour une protéine matricielle d'un premier rhabdovirus végétal et une deuxième séquence nucléotidique codant pour un polypeptide de surface, où le polypeptide de surface comprend
(i) une partie exposée en surface d'un polypeptide cible, où le polypeptide cible provient d'un virus animal, d'une bactérie, d'un parasite ou d'un mycète et où la partie exposée en surface du polypeptide de surface est antigénique
(ii) un domaine transmembranaire et
(iii) une queue cytosolique dérivée d'une protéine transmembranaire d'un deuxième rhabdovirus végétal ; et
(b) le maintien de la cellule végétale, de la plante ou de la partie d'une plante dans des conditions permettant la co-expression de la protéine matricielle et du polypeptide de surface, produisant ainsi les pseudo-particules virales.

5. Méthode de la revendication 4, où le premier rhabdovirus végétal et le deuxième rhabdovirus végétal sont les mêmes.

6. Méthode de la revendication 4, où au moins 50 % des pseudo-particules virales ont un diamètre qui est inférieur à 50 % du diamètre moyen de l'ensemble des pseudo-particules virales.

7. Méthode de la revendication 4, qui comprend en outre
(c) la purification des pseudo-particules virales.

8. Composition immunogène comprenant une quantité efficace des pseudo-particules virales de la revendication 1, où au moins 50 % des pseudo-particules virales ont un diamètre qui est inférieur à 50 % du diamètre moyen de l'ensemble des pseudo-particules virales.

9. Composition immunogène comprenant une quantité efficace des pseudo-particules virales produites par la méthode de la revendication 4, où au moins 50 % des pseudo-particules virales ont un diamètre qui est inférieur à 50 % du diamètre moyen de l'ensemble des pseudo-particules virales.

10. Pseudo-particule virale de la revendication 1 ou pseudo-particule virale produite par la méthode de la revendication 4 pour une utilisation en tant que médicament, de préférence pour l'induction d'une réponse immunitaire protectrice contre un agent pathogène chez un sujet.

11. Cellule végétale recombinante comprenant une ou plusieurs molécules d'acide nucléique, où les une ou plusieurs molécules d'acide nucléique comprennent une première séquence nucléotidique codant pour une protéine matricielle d'un premier rhabdovirus et une deuxième séquence nucléotidique codant pour un polypeptide de surface, et où le polypeptide de surface comprend
(a) une partie exposée en surface d'un polypeptide cible, où le polypeptide cible provient d'un virus animal, d'une bactérie, d'un parasite ou d'un mycète et où la partie exposée en surface du polypeptide de surface est antigénique
(b) un domaine transmembranaire et
(c) une queue cytosolique d'une glycoprotéine d'un deuxième rhabdovirus végétal.

12. Cellule végétale recombinante de la revendication 12, où le premier rhabdovirus végétal et le deuxième rhabdovirus végétal sont les mêmes.

13. Plante ou partie de celle-ci comprenant la cellule végétale de la revendication 11 ou 12.
